(19) Europäisches Patentamt
European Patent Office
Office européen des brevets

(11) **EP 1 958 588 A2**

(12) **EUROPEAN PATENT APPLICATION**

(43) Date of publication:
20.08.2008 Bulletin 2008/34

(51) Int Cl.:
*A61B 19/00* [(2006.01)]

(21) Application number: 08151511.6

(22) Date of filing: 15.02.2008

(84) Designated Contracting States:
AT BE BG CH CY CZ DE DK EE ES FI FR GB GR
HR HU IE IS IT LI LT LU LV MC MT NL NO PL PT
RO SE SI SK TR
Designated Extension States:
AL BA MK RS

(30) Priority: 19.02.2007 SE 0700406

(71) Applicant: RADI MEDICAL SYSTEMS AB
754 50 Uppsala (SE)

(72) Inventors:
• Eklund, Ann-Charlotte
753 30, Uppsala (SE)
• Tiensuu, Anna-Lisa
753 52, Uppsala (SE)
• Nicklasson, Eva
756 52, Uppsala (SE)

(74) Representative: Holmberg, Nils Anders Patrik et al
BRANN AB
Västgötagatan 2
P.O. Box 17192
104 62 Stockholm (SE)

(54) **Medical guide for guiding a medical instrument**

(57) A medical guide (1, 15) is provided for guiding a medical instrument into a target site within a patient's body, for use in connection with a cross-sectional imaging technique such as magnetic resonance imaging or computed tomography. The medical guide comprises a base (2,16) adapted to be positioned on the patient's body, a trajectory guide (5, 17) movably connected to the base, wherein the trajectory guide is provided with at least one distal marker (7, 22) and at least one proximal marker (8, 23) visible by said cross-sectional imaging technique, and the markers are separated by at least one portion not visible in a chosen cross-sectional imaging technique, Furthermore, the alignment markers are placed so as to optimize the trajectory path and facilitate the procedure of e.g. taking a biopsy sample using the selected cross-sectional imaging technique

Fig. 1

**Description**

**Field of the invention**

The present invention relates generally to a guide for guiding a medical instrument to a target site within a patient's body, and more particularly to a trajectory guide, visible using magnetic resonance imaging or x-ray based computed tomography (CT), for guiding a medical instrument, e.g. a biopsy instrument, to a target site, the position of which has been determined by means of magnetic resonance imaging or x-ray based computed tomography.

**Background of the invention**

Acquiring and providing diagnostic images of internal body structures and diseased or altered tissue is of great importance in many areas of medicine. Techniques to acquire such images include X-ray computed tomography (CT), ultrasonic imaging, emission tomography and magnetic resonance imaging (MRI). MRI and CT provide two-dimensional cross-sectional images through a patient, using magnetic fields and X-rays, respectively, visualizing internal structures in color or gray scale. These sections can be combined to visualize three-dimensional structures on or within a body. One advantage of using magnetic resonance imaging is that it does not expose the patient to harmful radiation while at the same time producing detailed images of the area of interest X-ray based computed tomography, on the other hand, is a more economical choice, and therefore more widely available.

When performing interventions such as e.g. biopsies or treatment of diseased or altered tissue, it is of utmost importance to plan the intervention carefully by choosing the entry point and trajectory path in order to reach the location of the desired site with minimum risk for the patient. By using for example an MRI scanner or a CT scanner, the physician is provided with images from which he or she can plan the procedure by choosing the entry point, the direction and the depth for an interventional device so as to reach the target site. Occasionally some kind of guiding device is used; however, in most cases, the puncturing device is manipulated without employing any device for ensuring the accuracy of the puncturing needle reaching the target.

A variety of guides employed to properly position a medical instrument within the body of a patient are known in the state of the art. US4608977 and US4733661 disclose guidance devices for use in conjunction with a CT scanner. US5263956 shows a ball joint for holding a neurosurgery tool in a predetermined orientation relative to a patient's skull. Devices used in stereotactic surgery are disclosed in US6110182 and US4805615. Notably, the abovementioned devices are not optimally adapted for use in conjunction with any simultaneous imaging techniques, and rely mostly on the judgment of the physician in determining the trajectory of the introduced medical instrument.

US6206890 shows a trajectory guide apparatus for use in introducing surgical and observational instruments into a patient's brain. The apparatus comprises a base plate, a movable guide member mounted as a ball joint and, in the case of using computed tomography or magnetic resonance imaging, a separate guide rod with two locator markers, visible using the chosen imaging technique. However, US6206890 fails to indicate any design specifications concerning the two locator markers. Especially when locating small and/or deeply located lesions, accurate means to target the desired site are necessary. On the other hand, for practical reasons in a scanner environment, there is a limited space to maneuver the device.

**Summary of the invention**

One puncturing guide is disclosed in WO2004/021898 and US2004/0260312, assigned to the present assignee, and comprises a needle guide mounted on a base plate, where the needle guide is freely movable around a set entrance point on the patient's skin. The guide can therefore first be positioned and secured on the patient's body and thereafter manipulated to achieve the best entrance angle to reach the desired tissue site, without altering the entrance point. Using x-ray based CT, it has been found by the present assignee that by employing a metal needle guide, an artifact will in most cases be produced in the CT image, indicating the extended trajectory path into the tissue, even prior to puncturing the skin. The advantage of using a guide according to abovementioned patent applications in both magnetic resonance imaging and x-ray based computed tomography is its small size, as these techniques may sometimes limit access to the patient during the procedure. However, this puncturing guide is not visible in magnetic resonance imaging and is therefore of minimal use when planning the intervention with regards to optimizing the angle of the needle guide using magnetic resonance imaging.

Considering the practical limitations in both the field of view when performing a scan, and the physical restrictions of the scanner environment, and, on the other hand, simultaneously needing to provide sufficient accuracy for the trajectory, it is further desirable to optimize the distance between the alignment markers, in order to achieve a reliable trajectory path in each individual procedure.

A general object of the present invention is to provide a trajectory guide which can be used in a way that maximizes safety and minimizes patient discomfort during the intervention. Another object of the present invention is to provide a small and economical trajectory guide visible in magnetic resonance imaging and computed tomography for use in medical devices such as puncturing guides for biopsies and other surgical guides. A further object is to provide a method of using the trajectory guide to improve the accuracy when verifying the angle of entrance of a needle guide using magnetic resonance imaging or computed tomography prior to perforating the patient's skin and tissue.

The above-mentioned object is achieved by the present invention according to the preamble of claim 1 and provided with the features according to the characterizing portion of the independent claim, Preferred embodiments are set forth in the dependent claims.

Thus, an object is achieved by providing a specifically tailored trajectory guide as an aligning or guiding member of a medical device intended for introduction of instruments into the body of a patient The trajectory guide is provided with at least one distal and one proximal marker visible in magnetic resonance imaging or computed tomography. These markers are of such a size and distance from each other that they can be used to determine that the trajectory guide is dependably aligned with the target tissue. This is achieved by providing a function which can be used to determine the accuracy of the trajectory guide.

In a first embodiment the trajectory guide is provided as a hollow tube or instrument guide attached to a base which is attached or placed directly over the desired target tissue. The trajectory guide is freely angularly movable relative to the base and can therefore easily be aligned with the desired tissue site. In another embodiment, the trajectory guide is supplied as a separate member which can be attached to a trajectory guide having an instrument guide for a puncturing device, e.g. a biopsy needle. In a further embodiment, the trajectory guide is movable around a point that coincides with the defined puncturing entrance point. With such a puncturing guide, the tip of the puncturing device may be positioned at the puncturing point in a first operation and, in a subsequent operation; the entrance angle can be set without moving the position of the needle tip. In such an arrangement, the guide rod's distal marker is placed at the distal end of the rod, such that the distal tip of the guide rod is visible using magnetic resonance imaging and indicates the exact entrance point of the puncturing device.

## Brief Description of the Drawings

Fig. 1 illustrates a first embodiment of the trajectory guide according to the present invention.

Fig. 2 illustrates a second embodiment of the trajectory guide according to the present invention.

Fig. 3 illustrates a third embodiment of the trajectory guide according to the present invention.

Fig. 4 illustrates a fourth embodiment of the trajectory guide according to the present invention,

Fig. 5 illustrates a fifth embodiment of the trajectory guide according to the present invention.

Fig. 6a and 6b illustrate the use of an extended rnd, e.g, a biopsy instrument, in an image of a body section.

Fig. 7 illustrates the out-of-plane deviation and the variables to consider when determining the accuracy of targeting a desired tissue site within a human body.

## Detailed Description of Preferred Embodiments

The following description will describe the embodiments mainly in connection to using magnetic resonance imaging for determining the trajectory path using the present invention. It should however be noted, that it is within the scope of the present invention to apply a corresponding approach to using x-ray computed tomography or any other cross-sectional imaging technique, with the appropriate changes in materials used, as is known in the state of the art.

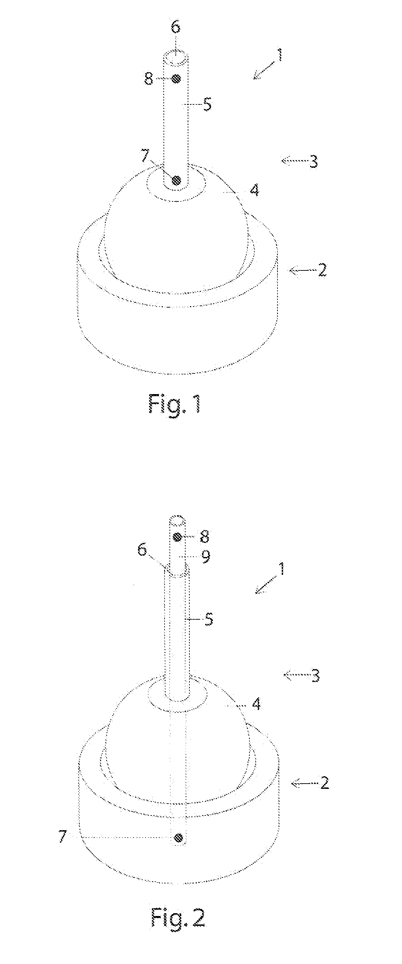

One embodiment of the trajectory guide 1 of the present invention (shown In Figure 1) comprises a base 2 which has a movable member 3 attached in such a way that the trajectory guide can be angled against the patient's skin in order to establish a trajectory path towards the tissue site of interest In this embodiment the movable member 3 is connected to the base 2 by a ball and socket joint, allowing the movable member to move freely over a wide range of angles and directions. The movable member comprises a ball 4 and attached elongated guide stem 5, through both of which a passage 6 forms part of the trajectory path. The guide stem 5 is preferably a hollow tubular member, adapted for introduction of a medical instrument such as a biopsy needle, a catheter, or another medical instrument. In order to visualize the trajectory path using magnetic resonance imaging the guide stem is provided with markers 7, 8 which are visible in the cross-sectional imaging technique used. These markers are separated by at least one segment which is either not visible in the imaging technique used or visually distinguishable from the markers. It is also possible within the scope of the present invention to provide three or several markers separated by segments visually distinguishable from the markers in the technique used, The use and placement of these markers will be further described below,

in another embodiment, illustrated in Figure 2, the medical guide is constructed in a similar way to that of Figure 1, with the exception of the markers on the guide stem 5. The markers 7, 8 are instead placed on a separate guide rod 9, which is introduced into the passage 6 of the guide stem 5. The guide stem is a hollow tubular member. After the trajectory path has been established, the guide rod 9 is removed and replaced by a medical instrument, such as a biopsy needle.

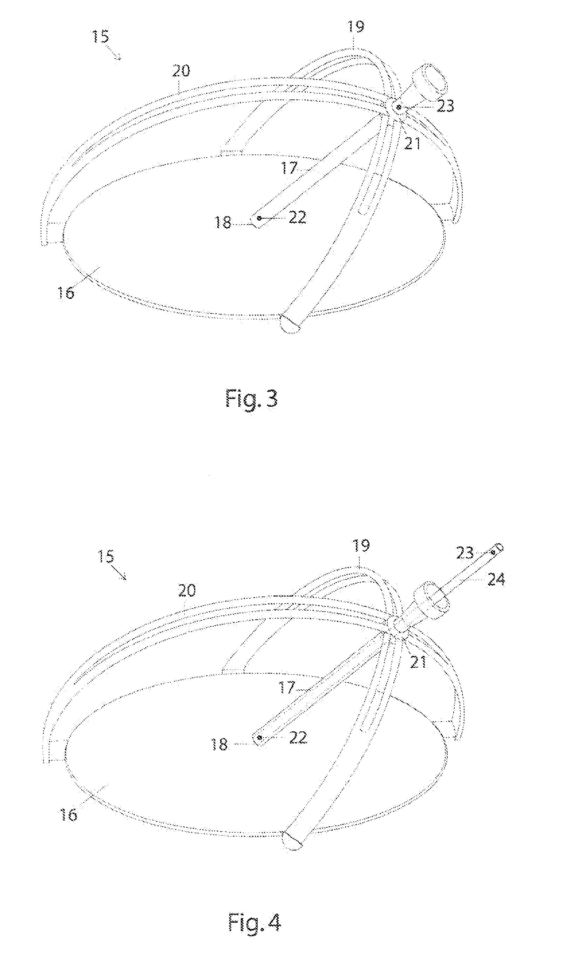

In a further embodiment shown in Figure 3, the medical guide of the present invention comprises a trajectory guide 15 for e.g. a biopsy needle, comprising a base plate 16 designed to be attached directly on the surface of the skin at the desired entrance point, and a movable guide member 17 attached in such a way that the trajectory guide is movable around a point 18 that coincides with the defined puncturing entrance point. This is achieved by arranging the movable guide member 17 in a first segment 19 of a first semi-sphere, and a second segment 20 of a second semi-sphere, and optionally providing a ring-shaped retainer 21.

The segments have the shapes of two semi-circular equally sized bows pivotally attached to the base plate 16 at positions separated by 90°, and a slit is provided in each bow, wherein the movable guide member 17 is adapted to be arranged in the slits at the intersection point of the bows and wherein the guide member 17 is movable around a point that coincides with the defined puncturing entrance point

The distal end of the trajectory guide 17 is introduced in a bore 18 in the center of the base plate 16. The inside of the ring-shaped retainer 21 is threaded and fits on a corresponding thread on the upper part of the guide member 17. Before the retainer 21 is tightened, the guide member 17 can slide inside the slits in the first and second segments 19, 20, with the distal tip of the guide member 17 being in contact, or almost in contact, with an object, such as the skin of a patient, beneath the base plate 16. The center of the first semi-sphere constitutes the rotational center of the guide member 17, which means that e.g. a puncturing needle introduced into the guide member 17 can be positioned in different angular orientations without changing the entrance point for the puncturing needle through a patient's skin. The retainer 21 is used to lock the guide in place after determining the optimal entrance angle.

Also in this embodiment the movable guide member 17 is provided with markers 22, 23 that are visible in the cross-sectional imaging technique used, Again, use and placement of these markers will be further described below, Similarly to the embodiment illustrated in Fig. 2, in yet another embodiment, the markers are placed on a separate guide rod 24 (Fig, 4).

In a third embodiment a combination of the previous embodiments is provided, such that at least one marker is located on the trajectory guide and at least one marker is located on the guide rod, The two markers should be spaced apart along the trajectory path such that they can be used in optimization of the trajectory angle, as will be described below.

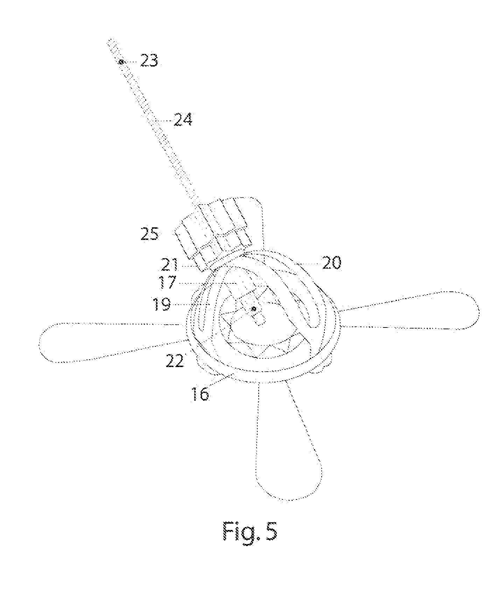

The detachable guide rod described above can preferably comprise a fastening means such as that illustrated in Figure 5, in order to attach the detachable guide rod 24 to the trajectory guide 17. Here the retainer 21 is provided with such a shape which enables secure fastening of a fastener 25, which in turn is integrated with the guide rod 24. in the illustrated embodiment, the fastener 25 grips retainer 21 by frictional forces. It should be noted that many other shapes are possible, as well as many other fastening mechanisms, such that guide rod 24 is securely fastened to the trajectory guide 17. Other possible fastening mechanisms include, but are not limited to, a snap-lock mechanism, corresponding threads on retainer and cap, and using screws. The guide rod itself can also be provided with external threads at the distal end, which are then screwed into matching internal threads in the hollow tubular guide member.

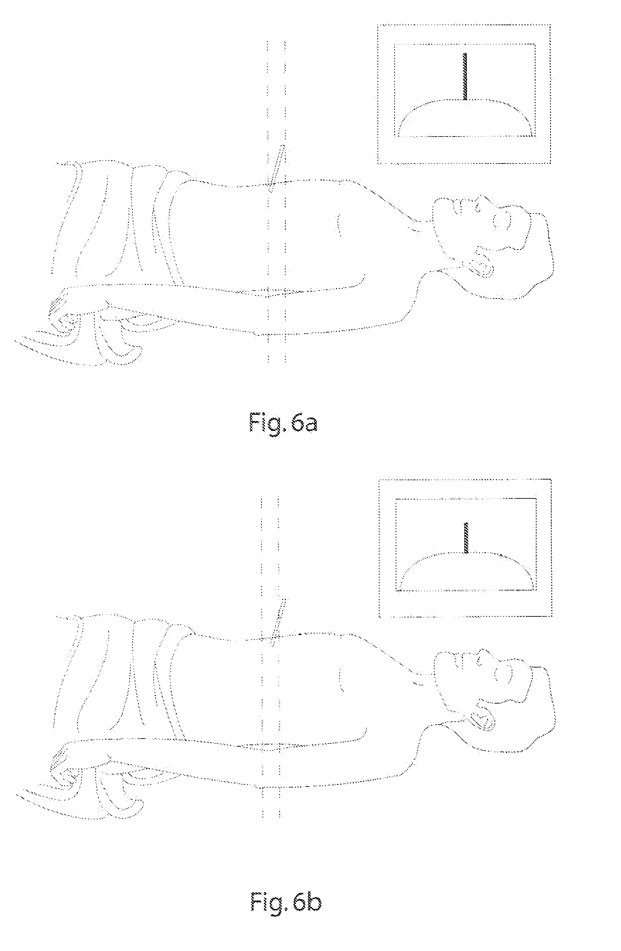

One unique feature of the present invention is the presence of argument markers in all embodiments. The alignment markers 7, 8, 22, 23 provide reference points in the images obtained by e.g. magnetic resonance imaging, which is further described below. When performing a biopsy or other surgical procedure requiring guiding an instrument to a specific site within a body, e.g, a tumor or other aberrant tissue, accurately determining the exact trajectory path prior to commencing the invasive procedure improves reliability and performance of the procedure, and can in many cases also reduce the time needed for the invasive part of the surgery, thereby reducing discomfort for the patient. In magnetic resonance imaging two-dimensional images are obtained, which represent virtual slices of a body section, The represented slices can be chosen to be at any angle in relation to the scanner or the body being depicted. These slices can commonly be between 1 mm and 2 cm thick. Consequently, all tissue visualized within this thickness will be depicted in one plane in the produced image. To determine whether a trajectory guide such as described herein is placed so as to allow access to the desired site within the body tissue, it is important to determine the precise angle of entrance into the body. In the obtained image, an in-plane angle of entry is easily calculated. However, as the two-dimensional image represents matter within a three-dimensional space, it can be difficult to visualize the out-of-plane offset angle. This is especially true using a trajectory guide consisting of a continuously visible elongated segment, as is illustrated in Figure 6. Here, the cross-sectional imaging apparatus is arranged to produce an image of a slice perpendicular to the length of the body, with a slice thickness indicated by the dotted lines. If a trajectory guide with approximately its entire length visible in the final image is slightly angled in out of the plane of the produced image it is possible that only part of the guide will be visible in the image, In such a case, it can be difficult to determine whether the guide visible in the image represents the entire visualized guide (see Figure 6a), or only a partial view (see Figure 6b), due to the fact that all material visualized within the chosen thickness will be depicted in one plane. Therefore, determining any offset angle out-of-plane is very difficult, if not impossible. By using separated markers as described herein and applying distinct calculations in planning the intervention, it is possible to guarantee that a sufficient part of the trajectory guide is within the chosen slice and, furthermore, more accurately establish that the trajectory path is actually angled correctly towards the desired tissue site. This will be further discussed in detail below.

The present inventors have realized that the uncertainty in determining the out-of-plane trajectory angle in relation to the target tissue can vary depending on the distance chosen between the two markers, Specifically, if a too small distance is chosen between the markers, and depending on the thickness of the body section chosen for imaging, it can be difficult to determine whether the entire rod is oriented entirety parallel to the surface plane of a particular depleted cross-section. On the other hand, a very large distance between the two markers is not practical, due to the limited actual space within the scanner environment and the need to limit the field of view for computational reasons. In addition, as the patient is not absolutely still during a scanning procedure due to breathing etc, it would be very difficult to keep two distantly spaced markers within the same slice for any longer period of time. Especially when performing procedures where the targeted tissue is of a smaller size, it is crucial to be able to accurately determine the trajectory path in three-dimensional space.

The high accuracy is achieved in the present invention by constructing markers that are sufficiently separated from each other on the trajectory guide to be able to determine a deviation from the image plane.

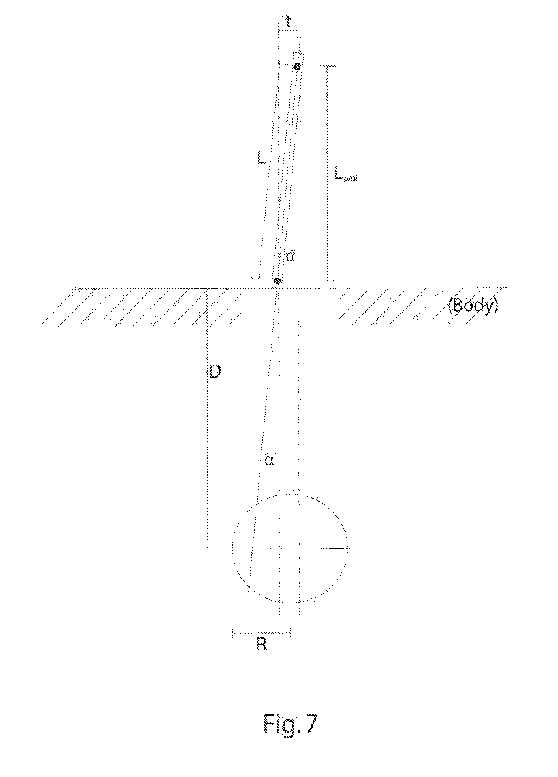

To be useful as a trajectory guide, both the two markers need to be visible in the produced image. This is illustrated in Figure 7. Consequently, the minimum distance between these two markers is defined as the length $L_{min}$, and is represented by the equation

$$L_{min} = t/(\sin \alpha_{max}) = t/(\sin(\arctan (R/D))) \qquad (1)$$

wherein D is the depth of the lesion measured from the entrance point to the center of the lesion, and t is the thickness of the slice depicted in the image. R is the radius of the targeted tissue mass, e.g. a lesion, or, in practice, approximately half the extension of the lesion as estimated or measured in a plane perpendicular to the image plane, and $\alpha_{max}$ is the maximum allowed deviation angle from the image plane in order to still target a lesion of size 2R. There are some practical aspects of the imaging technique that need to be considered. As a person skilled in the art will know, the thickness, t, of the slice depicted is generally chosen such that the target radius, i.e. R, is larger than or equal to t. The thickness is typically 3-5 mm. The image plane is preferably located approximately over the center of the lesion. It is also assumed that the signal from a single marker will weaken substantially as more than half of the marker is outside the image plane,

Referring to Fig. 7, equation (1) above is derived using the following geometric expressions:

$$\tan \alpha_{max} = (\sin \alpha_{max})/(\cos \alpha_{max}) = R/D \qquad (2)$$

$$\alpha_{max} = \arctan (R/D) \qquad (3)$$

$$L_{min} = t/(\sin \alpha_{max}) \qquad (4)$$

Notably, the two parameters D and R are available to the user in the MRI image, who can thereby calculate $\alpha_{max}$.

Furthermore, the projected distance between the markers, $L_{proj}$, can also be obtained from the image. Referring to Fig. 7, the lowing relationships are also valid:

$$L_{proj} = L_{min}{}^*\cos \alpha_{max} \qquad (5)$$

$$t/L_{proj} = \tan \alpha_{max} \qquad (6)$$

For very small angles, as is common in MRI, $L_{proj}$ will approximately equal $L_{min}$, and is such cases equation (1) and (5) can be approximated to

$$L_{min} = t*D/R \tag{7}$$

Therefore, for deeply located small lesions, i.e. where R is approximately equal to t and D is very much larger than t and R, the distance between the visible markers will have to be approximately the same as the depth of the lesion in order to obtain good accuracy of the intervention, For larger more shallow located lesions, equation (1) should be considered.

In practice, when the distance $L_{min}$ is known on the guide, the visible distance $L_{proj}$ in the image at a known t gives a reasonable estimate of whether the target of size 2R at depth D (in the image plane) will be accurately targeted using the trajectory guide at hand. This is illustrated in Example 1. By supplying tabular data there is also room for adjustment of the image plane thickness to reach a deseed accuracy.

Using the abovementioned equation and the empirical limitations observed by physicians in using e.g. magnetic resonance imaging when taking a biopsy, the markers are preferably placed at a distance of between 1 and 30 om from each other, and more preferably between 5 and 15 cm. The markers should also be small enough to each define a specific reference point in the image.

One solution to accurately targeting all lesions regardless of size and depth is to make a trajectory guide with two markers spaced very far apart, however, as stated above, this is not always practical. Therefore, in other embodiments, the trajectory guide is provided with three or more alignment markers, spaced along the trajectory guide in a straight line, in order to offer additional points of reference for the alignment. The number of markers is preferably between 2 and 20, more preferably 5 to 10. Using more than two markers provides the present invention with the means to determine the level of accuracy of reaching the desired target site, such that a given number of visible markers in an image plane of thickness t, compared to the total number of markers on the guide corresponds to a given accuracy (according to Example 2), This gives the physician the opportunity to assess whether it is advisable to attempt e.g. a biopsy, depending on for instance the size and/or depth of the target tissue, Furthermore, using additional markers could also provide the image with a measuring scale, which would ease the determination of e.g. the precise depth of the target tissue. It should be noted that in the case of providing the trajectory guide with three or more markers, the abovementioned length (L) in determining the accuracy of the trajectory guide represents the distance between the two outermost markers visible in the produced image.

In a further embodiment, at least one marker is mounted on the trajectory guide such that it is movable along the length of the trajectory guide. This adds the advantage of being able to adapt a trajectory guide to specific situations wherein a higher accuracy is desired.

As mentioned above, in some cases, specific constraints arise in individual procedures which limit the physical space available for a medical device. An example of such a constraint is when either the field of view or the actual physical space available in the scanner does not allow the placement of a long guide rod. Therefore, an additional embodiment provides a detachable guide rod comprising at least one proximal section which can be disengaged or broken off, to allow a shorter guide rod to be used. The shortest possible guide rod to be used still composes at least two markers.

When the present invention is to be used in connection with either magnetic resonance imaging or computed tomography the markers comprise a contrast medium chosen among those common in the state of the art. The contrast medium for computed tomography can comprise, but is not limited to barium sulfate and iodine-based compounds. The contrast medium for magnetic resonance imaging can comprise, but is not limited to, water, lipids, formulations based on gadolinium, dysprosium, manganese or iron. Considering the chosen imaging technique, the contrast medium can either be present as a liquid, a gel or in solid form. In the first two cases, the substance is enclosed in a capsule or similar container, which is then attached to or encapsulated within the trajectory guide. The markers can be incorporated within the material of the guide member or the guide rod. Additionally, the markers can be produced separately from the trajectory guide and attached to the trajectory guide, either detachably or permanently affixed to the guide member or the guide rod.

All the components of the present invention, other than the alignment markers, should comprise materials which are either not visible in computed tomography and magnetic resonance imaging, or should be visible in such a way that the markers are easily distinguishable in the produced image, The choice of materials in instruments and medical devices is important when using magnetic resonance imaging. All magnetic metals constitute a danger, as they will be pulled towards the magnet with great force. Several accidents have occurred when metal objects have injured the patient during the procedure. Non-magnetic metals do not pose a health hazard, but will quench the signal in an area surrounding the metal object. Therefore, all metals are avoided, With the above in mind, the trajectory guide may comprise components made of any materials known in the state of the art. Non-limiting examples of material which can be used in computed tomography and magnetic resonance imaging are carbon fiber, glass fiber, plastics or ceramics.

Although the present invention has been described with reference to specific embodiments it will be apparent for those skilled in the art that many variations and modifications can be performed within the scope of the invention as described in the specification and defined with reference to the claims below.

The invention is further described in the following non-restrictive examples.

**Example 1**

Given that

$$L_{min} = t/(\sin \alpha_{max}) = t/(\sin(\arctan (R/D))) \qquad (1)$$

a length between two markers on a trajectory guide of 10 cm, a lesion with a radius of 0.25 cm and a slice thickness of 0.2 cm, the lesion will be accurately targeted if it lies at a maximum depth of 12.5 cm.

**Example 2**

If five different markers are used, with equal distance between each marker along a straight line, and given that

$$L_{min} = t/(\sin \alpha_{max}) = t/(\sin(\arctan (R/D))) \qquad (1)$$

a lesion with a radius of 0.25 cm and a slice thickness of 0.2 cm, the indicated markers visible in the image obtained in Table 1 correspond to a maximum depth ($D_{max}$) where a lesion will be accurately targeted. Note that the markers are labeled $m_1$ through $m_5$, where $m_1$ is the most distal marker (i.e. essentially at the entrance point of e.g. the biopsy guide) and $m_5$ is the most proximal marker and the distance between $m_1$ and $m_5$ is equal to L.

**Table 1**

| Markers visible in image | $D_{max}$ | $D_{max}$ if L=10 cm |
|---|---|---|
| $m_1$ | 0.25*L | 2.5cm |
| $m_1$, $m_2$ | 0.5*L | 5.0 cm |
| $m_1$, $m_2$, $m_3$ | 0.75*L | 7.5cm |
| $m_1$, $m_2$, $m_3$, $m_4$ | 1.0* L | 10.0 cm |
| $m_1$, $m_2$, $m_3$, $m_4$, $m_5$ | 1.25*L | 12.5cm |

**Claims**

1. A medical guide (1, 15) for guiding a medical instrument through an entrance point to a target site within a patient's body, for use in connection with a cross-sectional imaging technique, comprising:

   a base (2, 16) adapted to be positioned on the patient's body,
   a trajectory guide (5, 17) movably connected to the base, the trajectory guide further comprising at least one distal marker (7, 22) and at least
   one proximal marker (8, 23) visible by said cross-sectional imaging technique, and the markers being separated by at least one portion visually distinguishable from the markers in said cross-sectional imaging technique,

   **characterized in that** the distance between said markers is determined by a function of the depth of the target site, the estimated radius of said target site and the thickness of a depicted body section.

2. A medical guide according to claim 1, **characterized in that** the function to calculate the distance (L) between said markers is represented by

$$L_{min} = t/(\sin(\arctan(R/D)))$$

or any approximation thereof, wherein D is the depth of the target site measured from the entrance point to the center of the target site, t is the thickness of a depicted body section, and R is the estimated radius of the target site.

**3.** A medical guide according to claim 1, **characterized in that** said trajectory guide comprises up to twenty markers visually distinguishable in said cross-sectional imaging technique, and said markers are each separated by at least one portion visually distinguishable from the markers in said cross-sectional imaging technique.

**4.** A medical guide according to claim 3, **characterized in that** said markers are interspaced such that a given number of the total number of markers corresponds to a given accuracy of reaching the target site,

**5.** A medical guide according to claim 1, **characterized in that** said trajectory guide comprises a hollow tubular guide for a medical instrument

**6.** A medical guide according to claim 1, **characterized in that** said trajectory guide comprises a hollow tubular guide and a detachable guide rod (9, 24) adapted to be arranged in said hollow tubular guide, wherein at least one of said markers is located on the guide rod.

**7.** A medical guide according to claim 6, **characterized in that** said guide rod further comprises a mechanism to detachably attach to the hollow tubular guide.

**8.** A medical guide according to claim 6, **characterized in that** at least one of said markers is movable along the length of the guide rod.

**9.** A medical guide according to claim 6, **characterized in that** the guide rod comprises a mechanism to disengage at least one proximal section.

**10.** A medical guide according to claim 1, **characterized in that** said cross-sectional imaging technique is magnetic resonance imaging.

**11.** A medical guide according to claim 1, **characterized in that** said cross-sectional imaging technique is computed tomographic imaging.

**12.** A medical guide according to claim 1, **characterized in that** said trajectory guide is movable around a point that coincides with a defined puncturing entrance point.

**13.** A medical guide according to claim 1, **characterized in that** said distal marker is placed in close proximity to a defined puncturing entrance point.

**14.** A medical guide according to claim 1, **characterized in that** said distal and proximal markers comprise contrast medium.

**15.** A medical guide according to claim 14, **characterized in that** said contrast medium comprises at least one of barium sulfate, iodine-based compounds, water, lipids, formulations based on gadolinium, dysprosium, manganese and iron.

**16.** A medical guide according to claim 14, **characterized in that** said contrast medium is incorporated in the material of the trajectory guide.

**17.** A medical guide according to claim 1, **characterized in that** at least one of said markers is movable along the length of the trajectory guide.

**18.** A medical guide according to claim 1, **characterized in that** said guide comprises at least one intermediate marker arranged between said distal market and said proximal marker, all markers being visible by said cross-sectional imaging technique, and the markers being separated by at least one portion visually distinguishable from the markers in said cross-sectional technique,

**19.** A guide rod for use in determining a trajectory path through an entrance point to a target site within a patient's body, in connection with a cross-sectional imaging technique, comprising;

at least one distal marker and at least one proximal marker visible by said cross-sectional imaging technique, and the markers being separated by at least one portion visually distinguishable from the markers in said cross-sectional imaging technique,

**characterized in that** the distance between said markers is determined by a function of the depth of the target site, the estimated radius of said target site and the thickness of a depicted body section.

**20.** A guide rod according to claim 19, **characterized in that** the function to calculate the distance (L) between said markers is represented by

$$L_{min} = t/(\sin(\arctan(R/D)))$$

or any approximation thereof, wherein D is the depth of the target site measured from the entrance point to the center of the target site, t is the thickness of a depicted body section, and R is the estimated radius of the target site.

Fig.1

Fig.2

Fig.3

Fig.4

Fig. 5

Fig.6a

Fig.6b

Fig.7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4608977 A
- US 4733661 A
- US 5263956 A
- US 6110182 A

- US 4805615 A
- US 6206890 B
- WO 2004021898 A
- US 20040260312 A